# EUROPEAN PATENT APPLICATION

(11) **EP 3 552 537 A1**
(43) Date of publication of application: **16.10.2019**
(21) Application number: 17880295.5
(22) Date of filing: 04.12.2017
(51) Int. Cl.: A61B 5/00, G05B 19/00

(54) **HEALTH DEVICE FOR SMART HOME CARE**

(30) Priority: 12.12.2016 CN 201611142151
(71) Applicant: Chen, Kaipo, Taoyuan Hsien 32571 (TW)
(72) Inventor: Chen, Kaipo, Taoyuan Hsien 32571 (TW)
(74) Representative: Horak, Michael
(86) International application number: PCT/CN2017/114439
(87) International publication number: WO 2018/107992

(57) **Abstract**

A smart home-care health device 1 includes a health device 1 including a master control board 11 for household application. The master control board 11 is electrically connected with multiple modules arranged in the health device 1. The detection modules 14 connected to the master control board 11 include a blood glucose detection module, a sleep detection module 146, and a heart detection module 147, which allow for acquisition of detailed and correct data of a target body 2 through non-contact detections and allows, through feed-back, transmission to the master control board 11 for judgment in order to establish personal dedicated message corresponding to the status of the target body 2 and allow for message transmission to a clinic or a hospital and electrical appliances 3 to allow family members to receive perfect home care of smart home.

## Description

### (a) Technical Field of the Invention

The present invention relates generally to a smart home-care lighting apparatus, in which an image pickup module capable of recognizing a target in a range of 360 degrees is involved in order to integrate a function of environment detection condition and allows for using lighting and sound for alarming and indication to provide better protection to the living of family members.

### (b) Description of the Prior Art

Existing smart home systems generally comprises a central processor host device for control purposes and multiple sensors, which require control processes that are set up in advance to correspond to and connect with smart household electrical appliances of the same brands. Thus, except those smart household electrical appliances that have been set up in advance, products of other brands or those of old models may not meet the need of integrated application desired by users so that only the brands that are supported by the suppliers may be purchases. Thus, it is quite an expense if a user wishes to experience such a system.

To solve the issues of low support and high expense of the known smart home-care system, control devices with easy operations have been proposed by manufacturers so that through the known communication technology including WIFI, Zigbee, Bluetooth, and the likes, connection can be made with corresponding smart household electrical appliances for operation, in combination with transducers mounted in the control devices in combination with detection made with surroundings and persons nearby, the un-supporting among brands can be overcome to allow a user to experience the convenience of smart home with the existing household electrical appliances.

However, in addition to the function of connection with household electrical appliances for driving, the smart home system have undergone development to such an extent of detecting the status of a user in order to determine physical function and be adapted to operation of electrical appliance; however, additional sensors must be worn, or use be made with connection through smart phone. In addition, the wearable device may be easily lost and damaged for children, adults, or elders.

In view of the above, the present invention aims to provide an improved structure in respect of detectability, range of application, and connection and controllability of the smart home systems.

### SUMMARY OF THE INVENTION

The present invention provides a smart home-care health device, which allows for easy installation of modules of different function at the manufacture side to provide a product with flexibility of adjustment, and also allows for maintenance of consistency of outside appearance in subsequent function updating or enhancing, making it more convenient in practical uses.

The present invention provides a smart home-care health device, which is adapted to conduct default operation for at least one or more than one target body and electrical appliance, comprising: a health device, which is provided therein with a master control board that receives and is driven by an input of electrical power, the master control board being connected to an image-taking module for taking an image of the target body for identify recognition, a communication module that supports remote control and transmits default linking of the master control board, a voice recognition module that is used in combination with the master control board to drive the electrical appliance, a detection module that detects data of the target body and environment, and a reminder module that is used in combination with the detection module; wherein the detection module is provided therein with a blood glucose detection module, a sleep detection module, and a heart detection module, for use with an electromagnetic radiation source to provide non-contact detection of physiological parameters of the target body in order to transmit to the master control board for conducting a corresponding handling operation.

The present invention is mainly such that emission of different frequencies from an electromagnetic radiation source to a target body in a non-contact manner is used to acquire data related to blood glucose, sleep, and heart of a target body and such data are analyzed through contents of individual databases used in combination with a master control board and artificial intelligence so that in any event of abnormality, connection is made to a clinic or a hospital or connection is made to surrounding electrical appliances associated therewith for automatic adjustment. When the conditions of the environment and the target body undergo severe changes, a corresponding handling operation is conducted so that for families having young children or elder people that require home care, the present invention, when used in combination with advantages that provided by prior art devices, can allow a smart home-care health device of the present invention to be more practical concerning range of detection and applicant of intelligence. For better understanding of the technical solution that the present invention adopts to achieve the above objects and features, and the efficacy thereof, preferred embodiments will be described in the following, reference being had to the accompanying drawings.

The foregoing objectives and summary provide only a brief introduction to the present invention. To fully appreciate these and other objects of the present invention as well as the invention itself, all of which will become apparent to those skilled in the art, the following detailed description of the invention and the claims should be read in conjunction with the accompanying drawings. Throughout the specification and drawings identical reference numerals refer to identical or similar parts.

Many other advantages and features of the present invention will become manifest to those versed in the art upon making reference to the detailed description and the accompanying sheets of drawings in which a preferred structural embodiment incorporating the principles of the present invention is shown by way of illustrative example.

### BRIEF DESCRIPTION OF THE DRAWINGS

FIG. 1 is a schematic perspective view illustrating the structure of the present invention.
FIG. 2 is a schematic view of the present invention.
FIG. 3 is a cross-sectional view of the present invention.
FIG. 4 is a block diagram of the present invention.
FIG. 5 is a schematic view illustrating a second embodiment of the present invention.
FIG 6 is a schematic view illustrating a third embodiment of the present invention.
FIG 7 is a schematic view illustrating a fourth embodiment of the present invention.
FIG. 8 is a schematic view illustrating a fifth embodiment of the present invention.
FIG. 9 is a schematic view illustrating a sixth embodiment of the present invention.
FIG. 10 is a schematic view illustrating a seventh embodiment of the present invention.
FIG. 11 is a schematic view illustrating an eighth embodiment of the present invention.
FIG. 12 is a schematic view illustrating a ninth embodiment of the present invention.
FIG. 13 is a schematic view illustrating a tenth embodiment of the present invention.
FIG. 14 is a schematic view illustrating an eleventh embodiment of the present invention.
FIG. 15 is a block diagram of the eleventh embodiment of the present invention.
FIG. 16 is a schematic view illustrating a twelfth embodiment of the present invention.

### DETAILED DESCRIPTION OF THE PREFERRED EMBODIMENTS

The following descriptions are exemplary embodiments only, and are not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention as set forth in the appended claims.

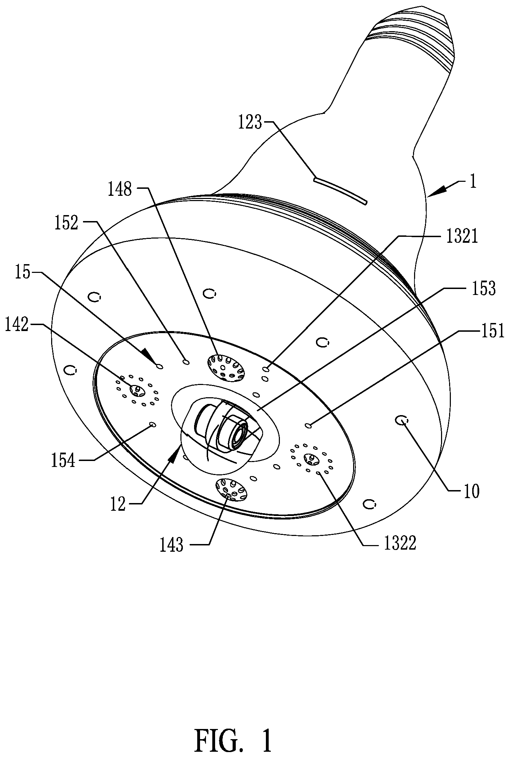

Generally according to the present invention, the best practicable embodiment, in combination with the drawings, a detailed description will be provided for better understanding of the present invention. The present invention provides a smart home-care health device, which, referring to FIGS. 1-4, comprises: a health device 1, which is mainly used to conduct presetting operations for at least one or more than one target body 2, and surrounding, corresponding electrical appliance 3, and the health device 1 is provided therein with, on the outside of a detection module 14, one or more than one LED 10 for application of lighting, wherein the LED 10 used in the present invention can be a single color LED or can be of a function of mixture and adjustment of color temperature, or can be constituted by single warm light, single cold light, or mixture of warm light and cold light, but not limited thereto.

The health device 1 has an outside appearance having a design similar to a common light bulb having a screw-to-lock structure and being provided therein with a master control board 11 that may receive an input of electrical power for driving and the master control board 11 is electrically connected with an image-taking module 12, a communication module 13, a detection module 14, and a reminder module 15. The image-taking module 12 is mainly used to take an image of the target body 2 for identity recognition and video recording and can be of dual-lens 121, or alternatively a fisheye lens 122 that maximizes a surveillance range, so as to ensure related linked functions can be normally operated. The image-taking module 12 is connected with a memory card slot 123 for storage of video recording.

The communication module 13 is for supporting remote control and transmission to the master control board 11 for perform default connection function, wherein the communication module 13 is connected with a microphone (1321) and a speaker (1322) and also comprises: a wireless control module 131, which can be wireless communication technology related to WIFI, Bluetooth, Z-WAVE, Zigbee or other wireless communication technology for connection with devices of multiple electrical appliances 3 or a handheld smart device (a tablet computer, a smart phone, a handheld or wearable device), a personal computer, and an in-vehicle infotainment system (IVI) so as to allow for remote management or operation and control of the electrical appliances 3. For old models of electrical appliances 3 of old models, they can be connected and used through the use of power extension cords or sockets with wireless connection, this being available in the market and repeated description being omitted herein. A voice recognition module 132 is operable to receive voice from the target body 2 for conversion into an instruction transmitted to the master control board 11 and the infrared control module 133. The voice recognition module 132 is connected with the microphone 1321 and the speaker 1322 for receiving and broadcasting sounds in order to achieve the purpose of voice control, whereby signals can be converted for transmission through infrared transceiver to each of the electrical appliances 3 to achieve the purpose of control.

The detection module 14 is operable for detecting data of the target body 2 and the surrounding environment and to be operable in combination with the reminder module 15 to provide alarm or indication. The detection module 14 comprises: a biological recognition module 141, which uses microwave to detect body features of the target body 2 for identity recognition and driving lighting and is often embodied with Doppler radar so that based on a moving signal of a high frequency band of a breath signal, when the breath signal is detected and it is found, based on the moving signal, that the target body 2 is moving in a fixed time, it is determined that the target body 2 is in an abnormal condition so that by using standing wave analysis, the detection range can be expanded and abnormality can be detected with high accuracy. A message of the detection can be fed back to the master control board 11 for recoding in Personal Health Record (PHD) so that abnormality can be transmitted, together with images and the detection message, to a hospital for Smart Medical Home (SMH) and telemedicine), or alternatively, in regular time, remote monitoring of family members, such as children and elders, can be conducted. The temperature and humidity detection module 142 is operable for detecting and feeding data of environment temperature and humidity back to the master control board 11, so that if the temperature and/or humidity is higher or lower than a predetermined threshold, corresponding ones of the electrical appliances 3 will be activated for operation. The smoke detection module 143 is operable for detecting if smoke in the environment exceeds a threshold in order to determine if there is an event of underside burning and fire. The smoke detection module 143 is electrically connected to the master control board 11 for transmission of signal. The air detection module 144 is operable for monitoring suspending particles in air and the air detection module 144 is connected to the master control board 11 and an air quality indicator 153 of the reminder module 15 in order to indicate the current status of air quality through lighting, such as blue indicating a normal range, yellow indicating an intermediate range, and red indicating exceeding a standard, wherein the displayed color can be adjusted through commonly known color mixture lighting technology of the LED 10, and may also be adjusted through an operation (application, APP) of a corresponding mobile phone and is not limited to the colors mentioned above and may allow displaying to be conducted with single crystal LED or polycrystalline LED, but is not limited to the techniques discussed herein. The gas detection module 148 is operable to detect if toxicant gases or other human-harmful gases exist in the environment and feed a signal back to the master control board 11 to conduct a corresponding handling operation, such as shutting down connected electrical appliances 3 and transmitting messages to user's mobile phones, in order to achieve an effect of prevention and protection.

The detection module 14 also comprises: a blood glucose detection module 145, a sleep detection module 146, and a heart detection module 147, these three detection modules being respectively provided with a blood glucose database 1451, a sleep database 1461, and a heart database 1471 that are independent of each other. Detected data are used to establish user information with respect to the associated database and are then fed back to artificial intelligence (AI) built in the master control board 11 for computation and determination and may be further analyzed and stored in order to improve accuracy of detection. The blood glucose detection module 145, the sleep detection module 146, and the heart detection module 147 all adopt ultra wideband (UWB) technology, using electromagnetic radiation sources 149 to emit different frequencies and intensities, to calculate data of incident waves and reflected waves so as to obtain data of different users for storage in all sorts of database. The characteristics of band expansion of the UWB technology allows for coexistence with other radio frequency (RF) system so that no mutual interference may occur, allowing application for detection range that has no dead zone. Through connection among multiple modules within the detection module 14, in combination with classification and analysis of the central processor 111 and the artificial intelligence 1111 of the master control board 11, user own data can be established for different users, making the use more convenient.

Further, the reminder module 15 of the present invention mainly comprises: a target body physiological signal indicator 151, which may display if physical condition of the target body 2 is normal or abnormal and determination is made according to data transmitted from the biological recognition module 141, the blood glucose detection module 145, the sleep detection module 146, and the heart detection module 147 to the master control board 11. The target body 2 can be users of different ages (children, adults, elders) or pets in the environment. A smoke/gas alarm light 152 is driven to display by a signal that the smoke detection module 143 transmits back to the master control board 11. An air quality indicator 153 is operable to display air quality of the environment detected by the air detection module 144. An IP setting indicator 154 may display if system operation and functions and setting are normal. All these indicators or lights are arranged inside the health device 1 as an arrangement of LEDs and allow for adjustment of displayed colors by means of mobile phones or computers through the master control board 11 and this provide a better effect of distinction than the prior art that uses single color displaying.

The communication module 13 of the present invention uses a wireless control module 131, a voice recognition module 132, and an infrared control module 133, and uses the artificial intelligence 1111 of the master control board 11 to recognize, in advance, an instruction regularly given by the target body 2 for preparing in advance related instructions or to first drive corresponding electrical appliances to be in a standby condition, allowing the connection with the electrical appliances to be more flexible.

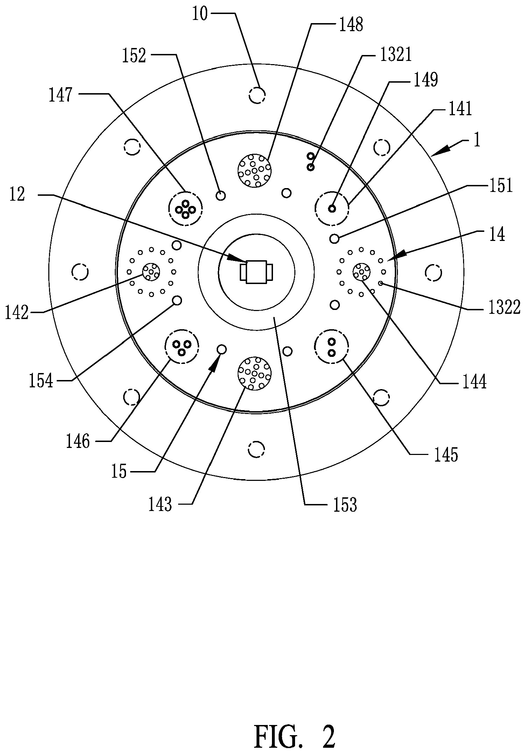

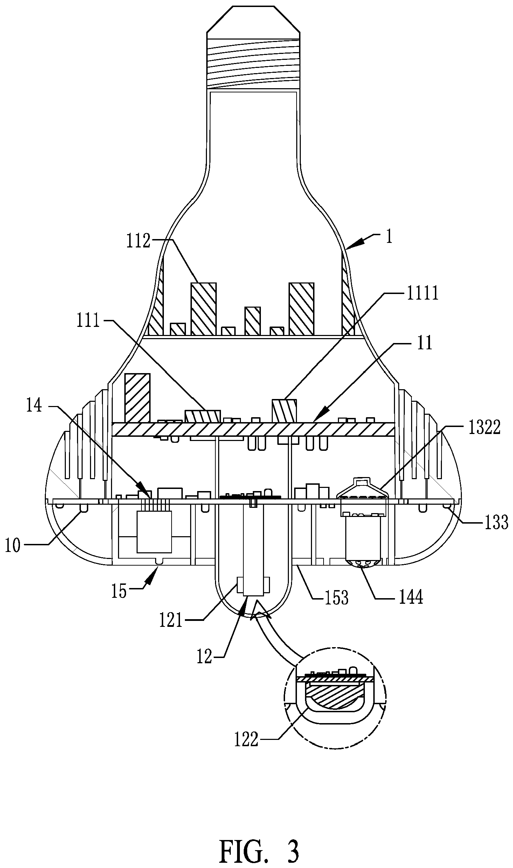

Referring to FIG. 2 again, the biological recognition module 141, the blood glucose detection module 145, the sleep detection module 146, and the heart detection module 146 of the present invention uses electromagnetic radiation source 149 of different intensities to conduct detection and can be made in a concealed design, in order to make the outside appearance look beautiful and also to protect the components/parts hereof from being influenced by external factors. As shown in FIG. 3, the speaker 1322 of the present invention is arranged at the back side of the detection module 14, allowing for effective reduction of space occupied and walls of predetermined thicknesses are present between the detection module to prevent mutual interference therebetween in conducting detection.

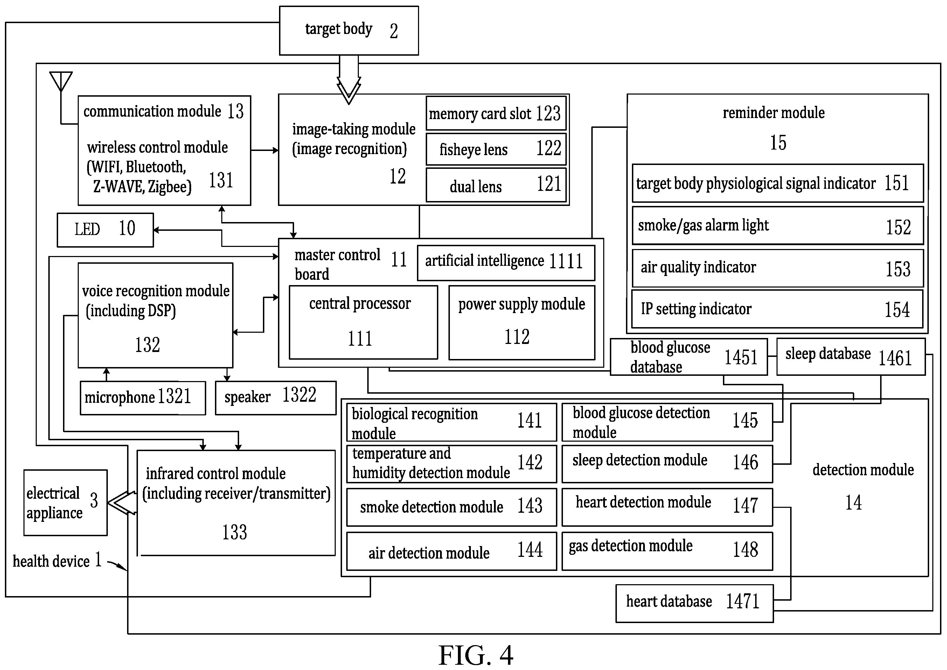

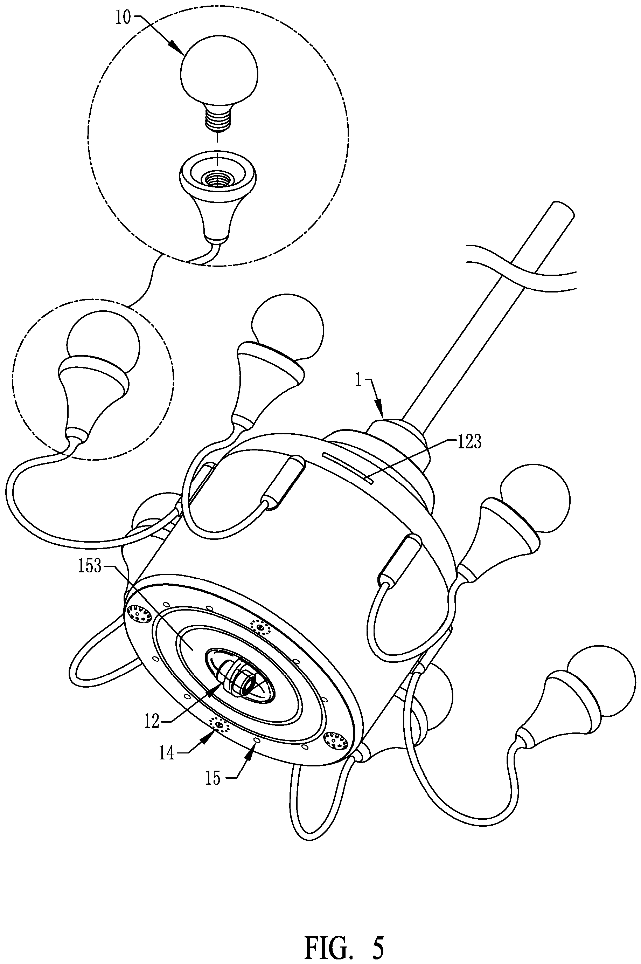

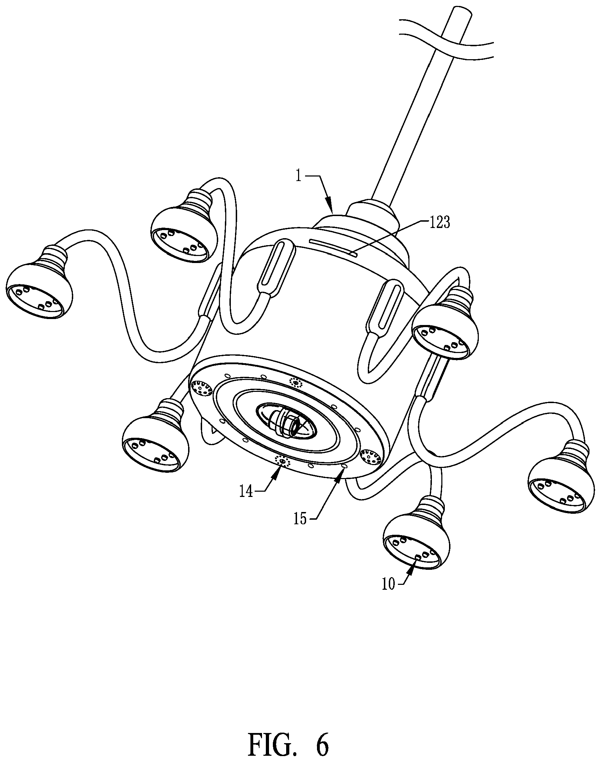

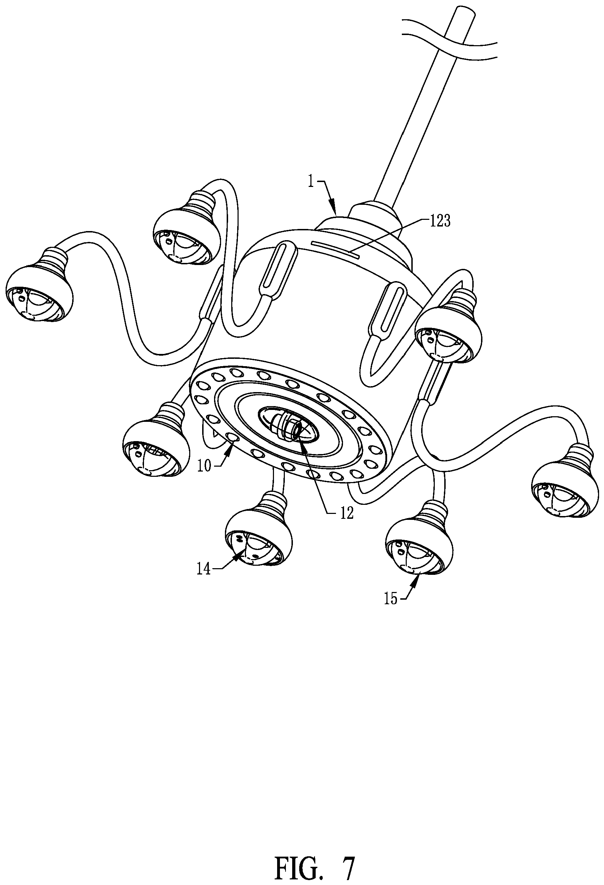

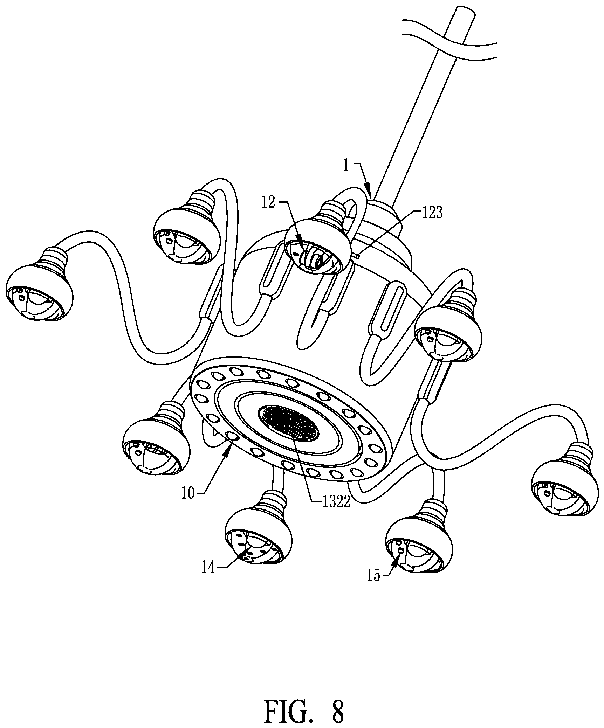

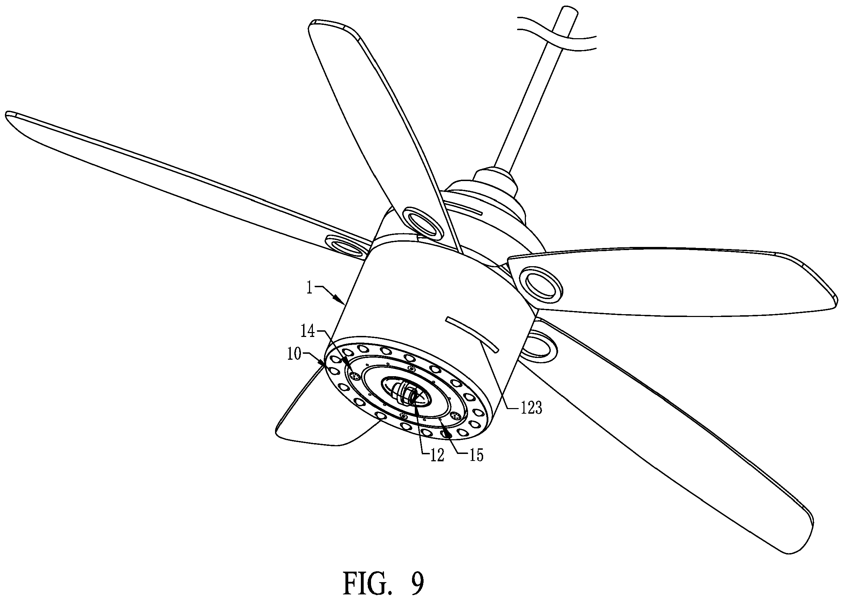

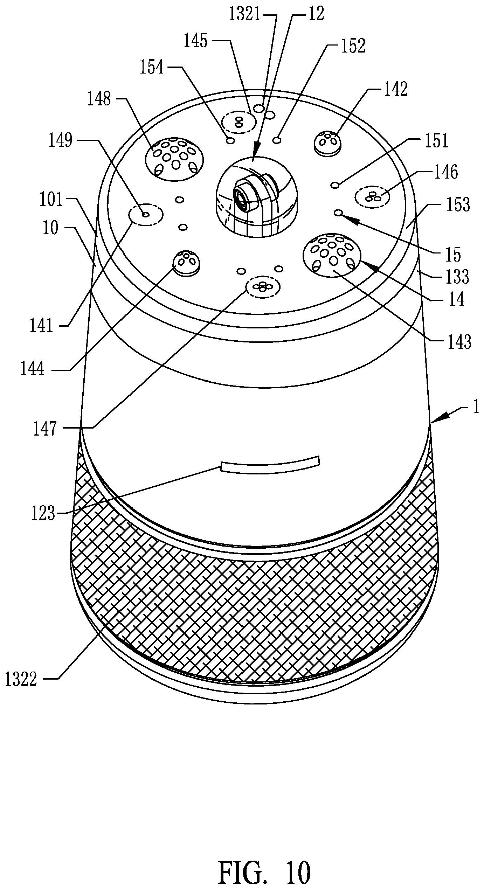

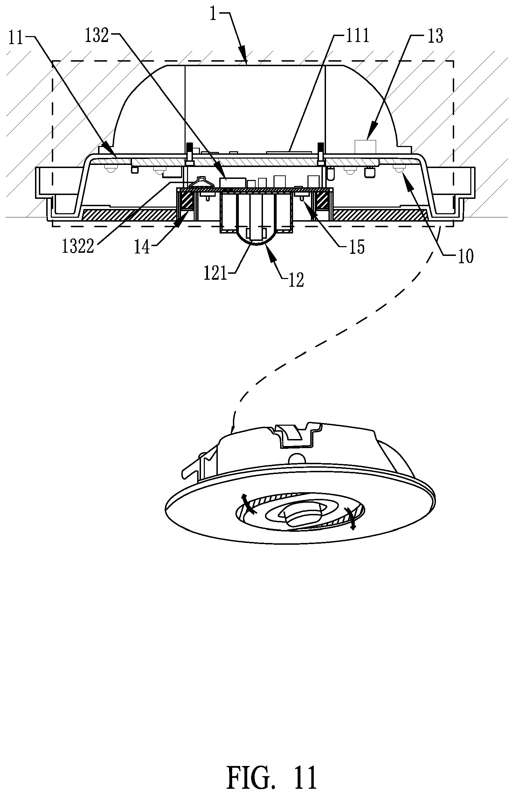

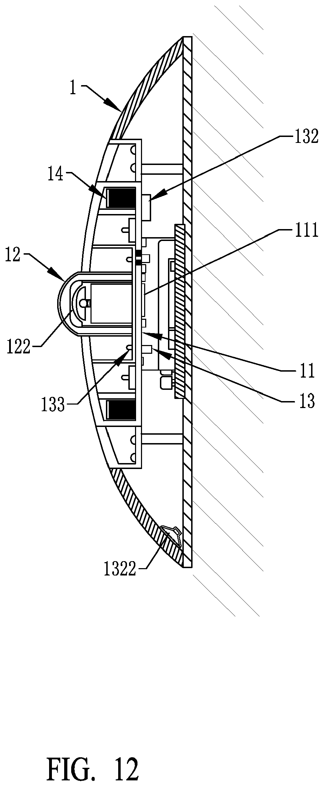

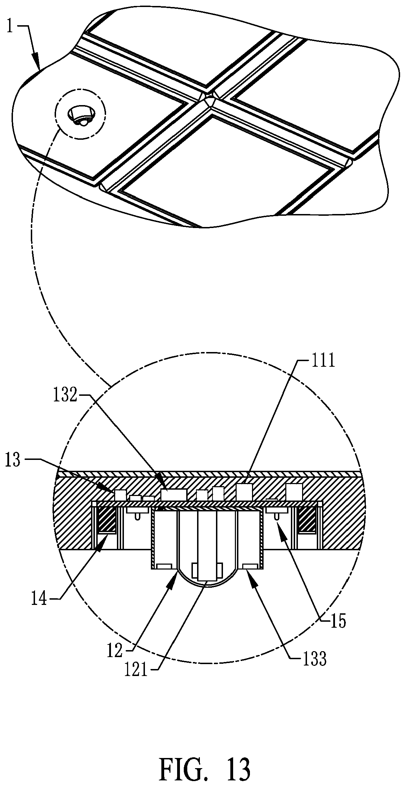

Referring to FIGS. 5-8, second to fifth embodiments of the present invention are shown, and are a pendant light structure, which comprises a central body and extension light assembly, and is generally structured such that the LED 10, the image-taking module 12, the detection module 14, and the reminder module 15 are arranged to have the positions of these modules exchanged with each other, while the purpose and function are similar to the operation flow illustrated in the block diagram of FIG. 4, but with only the positions changed to be fit to different light fixture. It can be seen from FIGS. 7 and 8 that the LED 10 is arranged at an inner side of the detection module 14, this being different from the illustration of FIG. 1 where the LED 10 is arranged at an outer side of the detection module 14, this indicating the position of the LED 10 can be changed to accommodate different designs. Further referring to FIG. 9, the pendant light shown in FIGS. 2-5 can be made in the form of a ceiling fan. Referring to FIGS. 10-13, a desk lamp, an embedded light, a wall lamp, and a flat fluorescent light are respectively shown and this indicates the present invention is applicable to various types of lights and lamps.

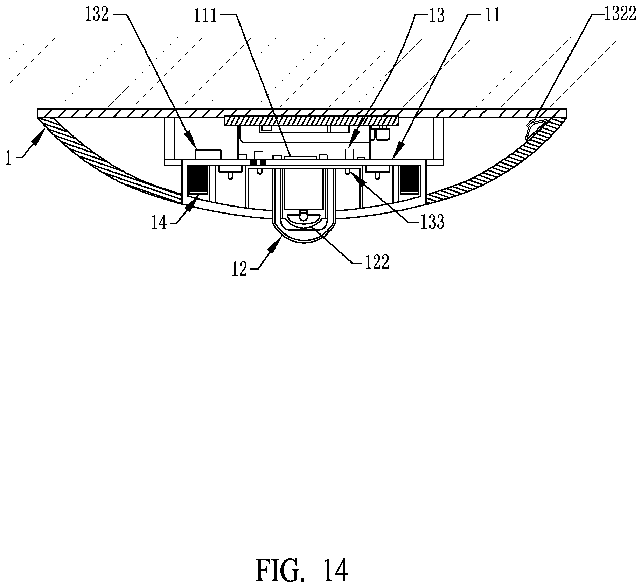

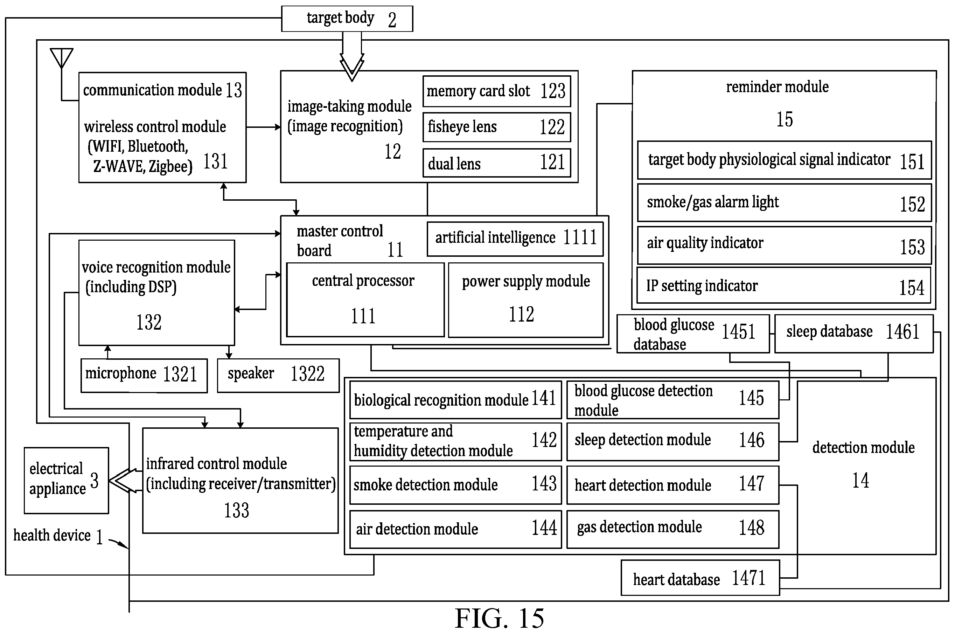

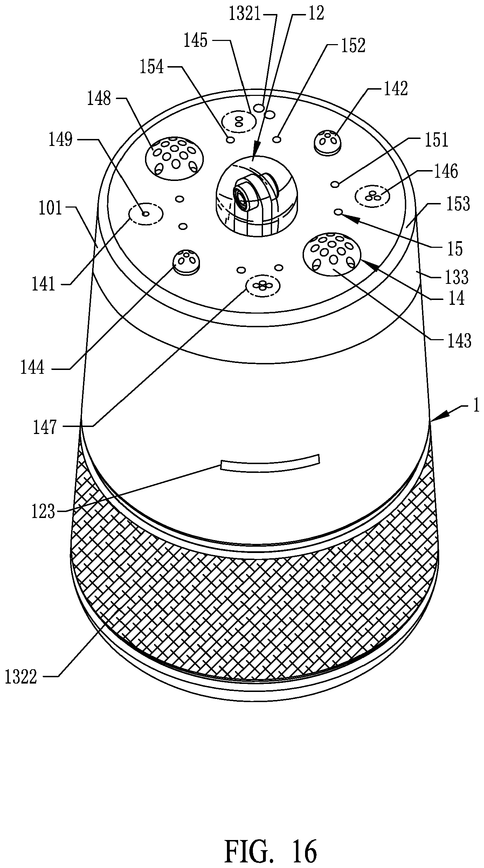

The health device 1 of the present invention can be structured simply for detection, or may be alternatively combined with an LED 10 for illumination and/or an UV-LED 101 having a function of disinfection, and is not limited thereto, so that it is not necessary to be made in the form of a light or a lamp. In addition, the master control board 11 and the infrared control module 133 arranged inside the health device 1 can be arranged as separate or combined as one or more one according to the actual specification and types manufactured. Referring to FIGS. 14-16, by removing the LED 10, modification can be made to a number of different designs, such as a ceiling light or a desk lamp, which still comprise the detection functions provided by the modules discussed above, this being an easy modification so that details will be omitted. The UV-LED 101 is embodied with ultraviolet C that has a relatively short wave length, this being a mature technique. The 8 modules of the detection module 14 can be added or removed according to the needs of manufacture and no limitation to a specific number is necessary.

In summary, the present invention provides a smart home-care health device, which comprises, installed in an interior thereof, multiple detection modules 14, which help improve accuracy of detection and allow for long term detection of physiological condition of a target body 3 in a non-contact manner and for abnormality prevention. The communication module 13 allows for easy connection with electrical appliances 3 and allows for enhancement of response for connection with the electrical appliances by means of the central processor 111 and the artificial intelligence 1111 of the master control board 1 and predict habits and favorites of the target body to establish corresponding connection and application, providing rich applicability of smart home.

It will be understood that each of the elements described above, or two or more together may also find a useful application in other types of methods differing from the type described above.

While certain novel features of this invention have been shown and described and are pointed out in the annexed claim, it is not intended to be limited to the details above, since it will be understood that various omissions, modifications, substitutions and changes in the forms and details of the device illustrated and in its operation can be made by those skilled in the art without departing in any way from the claims of the present invention.

## Claims

1. A smart home-care health device 1, which is adapted to conduct default operation for at least one or more than one target body 2 and electrical appliance 3, comprising:
a health device 1, which is provided therein with a master control board 11 that receives and is driven by an input of electrical power, the master control board 11 being connected to an image-taking module 12 for taking an image of the target body 2 for identify recognition, a communication module 13 that supports remote control and transmits default linking of the master control board 11, a voice recognition module 132 that is used in combination with the master control board 11 to drive the electrical appliance 3, a detection module 14 that detects data of the target body 2 and environment, and a reminder module 15 that is used in combination with the detection module 14;
wherein the detection module 14 is provided therein with a blood glucose detection module, a sleep detection module 146, and a heart detection module 147, for use with an electromagnetic radiation source to provide non-contact detection of physiological parameters of the target body 2 in order to transmit to the master control board 11 for conducting a corresponding handling operation.

2. The smart home-care health device 1 according to claim 1, wherein the detection module 14 further comprises: a blood glucose database 1451, a sleep database 1461, and a heart database 1471, the databases being such that through electromagnetic waves of different frequencies emitting from the electromagnetic radiation source to the target body 2 to acquire independent signal intensity for comparison and adjustment for transmission back to the master control board 11.

3. The smart home-care health device 1 according to claim 1, wherein the master control board 11 further comprises: a central processor 111 for transmitting an instruction for driving and connecting all modules; an artificial intelligence 1111 for analyzing and determining the blood glucose detection module, the sleep detection module 146, and the heart detection module 147 that have complicated data; and a power supply module 112, which allows for input of an external power supply or uses built in batteries to maintain stable operation.

4. The smart home-care health device 1 according to claim 1, wherein the reminder module 15 further comprises: a target body 2 physiological signal indicator 151, a smoke/gas alarm light 152, an air quality indicator 153, and an IP setting indicator 154, all the indicators and alarm lights being arranged in the interior of the health device 1 and electrically connected with the detection module 14, for displaying with single crystal or polycrystalline LED or LED 10 that lights with mixed color.

5. The smart home-care health device 1 according to claim 4, wherein the health device 1 further comprises: at least one or more than one LED 10 arranged at one side of the detection module 14 to allow the health device 1 to conduct lighting.

6. The smart home-care health device 1 according to claim 1, wherein the image-taking module 12 and the detection module 14 are selectively increased or decreased the number thereof by at least one or more than one.

7. The smart home-care health device 1 according to claim 1, wherein the detection module 14 further comprises:
a biological recognition module 141, a temperature and humidity detection module 142, a smoke detection module 143, an air detection module 144, and a gas detection module 148, all the detection modules 14 and recognition modules being arranged in the interior of the health device 1 and electrically connected with the master control board 11 to provide detection of corresponding function, allowing for increase or decrease of the number of all sorts of detection modules 14 and recognition modules.

8. The smart home-care health device 1 according to claim 1, wherein the master control board 11, the detection module 14, the image-taking module 12 are selectively increased or decreased the number thereof by at least one or more than one.

9. The smart home-care health device 1 according to claim 1, wherein the blood glucose detection module 145, the sleep detection module 146, and the heart detection module 147 inside the detection module 14 are selectively increased or decreased the number thereof by at least one or more than one.
